# NEW EUROPEAN PATENT SPECIFICATION

(11) **EP 3 056 187 B2**
(45) Date of publication and mention of the opposition decision: **30.08.2023**
(45) Mention of the grant of the patent: 22.08.2018
(21) Application number: 16154717.9
(22) Date of filing: 13.11.2012
(51) Int. Cl.: A61J 7/00, A61K 47/10, A61K 9/08, A61K 31/198, A61J 1/00

(54) **SINGLE-DOSE PHARMACEUTICAL PREPARATION OF THYROID HORMONES T3 AND/OR T4**
PHARMAZEUTISCHES EINZELDOSIS-PRÄPARAT AUS DEN SCHILDDRÜSENHORMONEN T3 UND/ODER T4
PRÉPARATION PHARMACEUTIQUE À DOSE UNIQUE D'HORMONES THYROÏDIENNES T3 ET/OU T4

(30) Priority: 14.11.2011 IT MI20112066
(43) Date of publication of application: 17.08.2016
(62) Divisional of application: 12798182.7
(73) Proprietor: Altergon S.A., 6900 Lugano (CH)
(72) Inventor: BELLORINI, Lorenzo, 6900 Lugano (CH); BERNAREGGI, Alberto, 6900 Lugano (CH); PIZZUTTI, Marco, 6900 Lugano (CH)
(74) Representative: Botti & Ferrari S.p.A.

(56) References cited:
- EP-A2- 1 291 021
- WO-A1-2010/086030
- US-A- 3 128 920

## Description

The present invention relates to a pharmaceutical preparation of thyroid hormones T3 and T4. Specifically, the invention relates to a pharmaceutical preparation of thyroid hormones T3 and T4 in water-alcohol solution, suitable for oral administration, provided in ready-to-use packaging, and characterized by high physical, chemical and microbiological stability. The invention also relates to said pharmaceutical preparation for use in the treatment of disorders caused by hormone T3 and/or T4 deficiency.

### BACKGROUND TO THE INVENTION

The thyroid hormones triiodothyronine or liothyronine (T3) and tetraiodothyronine or thyroxine (T4) are secreted by the follicular cells of the thyroid gland in response to the pituitary-gland hormone TSH, the production of which is regulated in turn by the hypothalamic hormone TRH. The secretion of thyroid hormones follows a circadian rhythm; the highest levels of T3 and T4 are reached during the night and the early hours of the morning.

T3 and T4 are essential for the normal body growth of children and for the maturation of the various apparatuses, especially the skeleton, and regulate metabolic activity in the adult, influencing the function of every organ and tissue. In particular, T3 and T4 increase oxygen consumption at rest, raising the basal metabolism, the body temperature and the daily calorie requirement. They regulate carbohydrate metabolism, promoting glycogenolysis and gluconeogenesis, and increase the activity of the enzymes involved in glucose oxidation. The thyroid hormones are involved in lipolysis and lipogenesis, regulate protein synthesis, exercising a trophic effect on the muscle, and affect the cardiovascular system.

Thyroid hormones are essential to the cardiac function: they increase myocardial contractility (positive inotropic effect), increase the heart rate (positive chronotropic effect) and increase venous return to the heart.

In general, the effect of the thyroid hormones is mainly anabolic at low doses, whereas they have a catabolic action at high doses. In situations of physiological deficiency of thyroid hormones, as in the case of primary and secondary hypothyroidism, a treatment based on T3 and/or T4 is required, administered as such or in the form of sodium salts or hydrates.

Treatment with thyroid hormones continues throughout the patient's life, and the posology (dose and frequency of administration) is customized according to the patient's response. The initial dose of T3 and T4 is usually low. The dose is then gradually increased until the clinical evaluation and laboratory tests indicate an optimum response. Selecting the dose is a critical aspect: an under-dose leads to a poor response, while an excessive dose can produce toxic symptoms of hyperthyroidism such as tachycardia, sweating, weight loss, nervousness, diarrhoea, bone resorption due to activation of the osteoclasts, and heart problems.

It is therefore important for patients to be able to count on reliable formulations in terms of dose accuracy.

Oral pharmaceutical forms of T3 and T4 hormones are on the market in solid form (regular tablets and soft gelatin capsules) and in liquid oral form in drops. This last pharmaceutical form makes the hormones immediately available for gastrointestinal absorption, and usually consists of a solution in a single multi-dose glass container with a dropper. The use of the dropper makes it difficult to measure accurately the volume of solution dispensed, and therefore the dose administered. If a T4 solution with a concentration of 100 µg/mL is used, for example, T4 doses of between 3.5 and 200 µg may be administered. Assuming that each drop (approx. 35 µL) contains 3.5 µg of T4, the administration of the maximum dose of 200 µg requires no less than 57 drops to be dispensed (2 mL). Moreover, the volume of the drops dispensed is not precisely replicable. The use of a dropper may therefore not guarantee the necessary dose accuracy, which is provided by other forms of administration such as tablets or soft capsules available in a variety of doses to allow optimum individual treatments.

The precarious accuracy of the dose represents a particularly important problem for drugs with a small therapeutic window such as thyroid hormones, especially in specific patient populations (children and the elderly). In the case of liquid pharmaceutical forms of T3 and/or T4, it is therefore evident that the availability on the market of single-dose pharmaceutical packs meets a clear therapeutic need.

In the liquid pharmaceutical forms (solutions, emulsions and suspensions) and semi-solid forms (creams, ointments and pastes), formulation ingredients for pharmaceutical use with high vapor pressure in general, such as alcohols, or inert gases, such as nitrogen, can act as solvents of the active ingredients present in the finished product or as preservatives, and can also, in synergy with other ingredients present in the pharmaceutical form, promote the physical and chemical stability of the finished product.

Volatile organic solvents such as ethanol are used to facilitate the dissolution of hormones T3 and/or T4. Adequate containment of volatile substances is necessary to guarantee the physical and chemical stability of the product. Evaporation of said substances from the formulation through the primary packaging material must be prevented, to avoid a gradual reduction in their concentration over time.

### STATE OF THE ART

Patent application WO2010/086030, filed by the applicant, discloses a single-dose squeezable container for solutions of T3/T4 hormones, which is made of plastic with a Young's modulus of between 10 and 80 MPa, and in particular of a mixture of polyethylene or polypropylene with ethylene vinyl acetate. However, it has been observed that although said container presents optimum squeezability characteristics which ensure that the solution is completely emptied, it is unable to preserve the chemical and physical stability of the water-alcohol solutions of hormones T3/T4 it contains, causing a loss of alcohol solvent and increasing the quantity of impurities present in it during storage.

The purpose of the present invention is therefore to overcome the drawbacks associated with the prior art, in particular the chemical and physical instability of water-alcohol solutions of hormone T3/T4 stored in the containers described in WO2010/086030, while maintaining the convenience and accuracy of dose and administration offered by single-dose squeezable containers.

### DESCRIPTION OF THE INVENTION

The present invention relates to a single-dose pharmaceutical preparation of thyroid hormones T3 and T4 suitable for oral administration, in ready-for-use packaging consisting of a container prefilled with a water-alcohol solution of hormone T3 and/or T4, said container being selected from:
(a) a one-component LDPE plastic container, squeezable by manual compression, with a Young's modulus of between 10 and 200 MPa, placed in a sealed sachet consisting of a laminated film made of different materials, in the following combination: polyethylene, aluminium and polyester;
(b) a multi-component laminated plastic container with a thickness of between 200 and 700 µm, squeezable by manual compression, having a Young's modulus of between 10 and 200 MPa, said container consisting of multiple layers of the following plastic materials: polyvinyl chloride, polyvinylidene chloride and polyethylene.

It is also described, but not part of the claimed invention, a sachet consisting of a film made of several layers of materials according to the following combinations: polyethylene, aluminium and polyester; polyethylene, aluminium and paper; ionomer resins, aluminium and paper.

In accordance with the invention, the claimed sachet can present oxygen and water vapor permeability preferably between 0.1 and 0.2 cc/m2/day.

In accordance with the invention, the water-alcohol solution can contain 1 to 20 µg of thyroid hormone T3 and/or 12.5 to 200 µg of thyroid hormone T4, ethyl alcohol in quantities preferably ranging between 5 and 30% w/v, glycerol in quantities preferably ranging between 70 and 95% w/v, and water.

At the end of the stability period, the pharmaceutical preparation according to the invention contains not less than 95% of the initial ethanol concentration, a T3 and/or T4 content of not less than 95% of the initial concentration, and total impurities (excluding impurity T3 in the single-dose T4 container) ≤ 2.5% of the initial concentration of active ingredient.

Moreover, the pharmaceutical preparation according to the invention, due to its characteristics, guarantees the absence of microbiological contamination, with TAMC (total aerobic microbial count) values ≤ 100 CFU/g, TYMC (total yeast and mould count) values ≤ 10 CFU/g, and absence of *E. coli,* thus being practical to use and not liable to accidental contamination.

The packaged pharmaceutical preparation according to the invention is for use to treat disorders associated with thyroid hormone T3 and/or T4 deficiency.

A further aspect of the invention therefore relates to the use of a water-alcohol solution of thyroid hormone T3 and/or T4 as defined above in a container as described above, to prepare a medicament for oral administration in the treatment of disorders associated with thyroid hormone T3 and/or T4 deficiency.

### DETAILED DESCRIPTION OF THE INVENTION

In the field of liquid oral formulations of hormones T3/T4, the choice of packaging material is important to allow the correct volume of solution to be delivered easy and precisely.

In order to be easily squeezable and ensure complete delivery of its contents, the container must be soft and malleable. This state is usually achieved with the use of low-density polyethylene, polypropylene and sometimes with the addition of suitable "softening" agents such as EVA, which produce "loose" cross-linking. Squeezable containers are characterized by a plastic material having a sufficiently low Young's modulus. The best results have been obtained with plastics characterized by a Young's modulus of less than 200 MPa. It has been demonstrated that when plastic containers with such a Young's modulus are used, the contents are almost completely extracted by a single manual compression.
The Young's modulus according to the purposes of the present invention is determined by: (1) generating a stress strain curve by subjecting specimens with the dimensions established by the applicable standard UNI EN ISO 527-1 (16.5 * 1.0 cm) to tension at the rate of 5 mm/min and (2) determining the slope of the initial, linear portion of the stress strain curve in which the Hook's law E = σ/ε is applicable, wherein:
σ = F/A where F is the force exerted on the specimen under tension and A is the cross-sectional area through which the force is applied;
ε = Δl/l where Δl is the amount by which the length of the object changes and 1 is the original length of the object.

Plastics with such a Young's modulus are obtainable by injection molding or blowing.

Known pharmaceutical-grade plastics are polyethylene (PE), ethylene vinyl alcohol copolymer resins (EVOH), polyvinyl chloride (PVC), polyvinylidene chloride (PVdC), polyvinyl acetate (PVA), fluorinated-chlorinated resins, ionomer resins, cyclic olefin copolymers (COC), polyamides (PA), polystyrene (PS), polycarbonate (PC), laminated metals such as aluminium bonded to plastics, blends of the materials listed above in variable percentages, or stratifications of the above-mentioned materials of variable thickness. However, although many of these materials produce containers that are easily squeezed and have low Young's modulus values, they have proved inadequate to guarantee the necessary physical and chemical stability of said formulation.

The best results have been obtained with an LDPE container packaged in a sachet made of non-breathable material as secondary packaging.

In terms of ethanol content, the physical stability of the T4 water-alcohol solution (100 µg/mL) in said single-dose container has proved significantly better than the stability of the same solution in single-dose primary packaging consisting of an EVA-PE blend (50%-50%, according to WO2010/086030) - see Table 1. The experiment was conducted by placing the samples at 40°C/75% RH for 7 days. The values are expressed as a percentage of the initial ethanol content.

**Table 1**

| 100 µg/mL T4 solution in single-dose containers stored at 40°C/75% RH for 7 days | | |
|---|---|---|
| **Prototype** | **Ethanol (%)** | |
| | T=0 | T=7 days |
| T4 solution in single-dose EVA-PE container | 99.8 | 88.69 |
| T4 solution in single-dose LDPE container in hermetically sealed sachet | | 97.80 |
| T4 solution in single-dose EVA-PE container in hermetically sealed sachet | | 91.90 |

The T4 preparation to which the present invention relates proved, in terms of total impurity content, more stable than the solution stored in single-dose primary packaging made of an EVA-PE blend (50%-50%) (Table 2).

The experiment was conducted by storing the samples at 50°C for ten days. The values are expressed as percentages of the initial active ingredient content.

**Table 2**

| 100 µg/mL T4 solution in single-dose containers stored at 50°C for 10 days | | |
|---|---|---|
| **Prototype** | **Total impurities (%)** | |
| | T=0 | T=10 days |
| T4 solution in single-dose LDPE container in hermetically sealed sachet | 0.7 | 2.70 |
| T4 solution in single-dose EVA-PE container | | 3.10 |

The T4 preparation according to the invention in single-dose packaging proved more chemically stable than similar formulations in multi-dose amber glass bottles present on the market.

Table 3 compares the values of the impurities after storage under ICH conditions at 25°C/60% RH for 6 months. The values are expressed as percentages of the active ingredient content.

**Table 3**

| 100 µg/mL T4 solution stored at 25°C/60% RH for 6 months | | |
|---|---|---|
| **Prototype** | **Total impurities (%)** | |
| | T=0 | T=6 months |
| T4 solution in single-dose LDPE container in hermetically sealed sachet | 0.7 | 1.87 |
| Commercial T4 solution in multi-dose glass bottle | 0.5 | 2.09 |

The chemical stability of the T4 solution, stored in packaging according to the present invention, proved better than that of the same solution stored in multi-dose amber bottles. Table 4 compares the values of the impurities in samples stored under ICH conditions at 25°C/60% RH for 6 months. The values are expressed as percentages of the initial active ingredient content.

**Table 4**

| 100 µg/mL T4 solution stored at 25°CI60% RH for 6 months | | |
|---|---|---|
| **Prototype** | **Total impurities (%)** | |
| | T=0 | T=6 months |
| T4 solution in single-dose LDPE container in hermetically sealed sachet | 0.7 | 1.87 |
| T4 solution in multi-dose glass bottle | | 2.82 |

In terms of ethanol content and total impurities, the physical and chemical stability of the water-alcohol solution of T4 (100 µg/mL) in a single-dose LDPE container in hermetically sealed sachet proved even greater than that packaged in a single-dose container made of SURLYN^{®} resins (code 1652) used for packaging in various fields, such as the food and cosmetic fields (Table 5).

**Table 5**

| Stability of the T4 100 µg/mL solution stored at 30°C/65% RH | | | | | | |
|---|---|---|---|---|---|---|
| **Prototype** | **Ethanol (%)** | | | **Total impurities** | | |
| | T=0 | T=30 | T = 3 | T=0 | T=30 | T = 3 |
| T4 solution in single-dose LDPE container in hermetically sealed sachet | 102.2 | NA | 101.5 | 0.7 | NA | 0.7 |
| Single-dose container made of SURLYN resin^{®} (code: 1652) | 99.8 | 92.0 | NA | 0.7 | 1.79 | NA |

Similar results were obtained with other SURLYN resins^{®}, such as SURLYN^{®} code 1802 and SURLYN^{®} code 1702. The containers made of SURLYN^{®} resins are not according to the claimed invention.

The extractability of the water-alcohol T4 solution from the single-dose LDPE container containing 1.1 mL of solution was evaluated by means of a squeezability test based on successive squeezes to demonstrate the complete, reproducible release of the expected volume of solution, namely 1.0 mL. The test involved a representative population of 60 subjects, matched for gender and age, divided into three groups consisting of 10 males and 10 females aged under 30 years, over 50 years or between 30 and 50 years. The subjects were asked to follow the procedure described below: *Hold the softer central part of the container firmly between the thumb and index finger. Applying firm pressure, squeeze the liquid into a glass, then release the container. Repeat this procedure 5 times.* The weight of the volume squeezed out by each squeeze was determined with an analytical balance. The weight was than converted to the corresponding volume using the density value (d) of the solution determined experimentally at ambient temperature (d=1,1145 g/mL).

The mean values of the volumes squeezed out are shown in Table 6, together with the standard deviation (SD), coefficient of variation (CV%) and 95% confidence interval (95% CI).

The results demonstrate that the volume of 1.0 mL of T4 solution is completely extracted from the containers if they are squeezed 3-4 times. This guarantees the administration of an accurate, reproducible dose to the patient. The volume extracted does not exceed the nominal value of 1 mL if the container is squeezed further.

**Table 6**

| Extractability of T4 solution from single-dose LDPE containers | | | | | |
|---|---|---|---|---|---|
| Solution T4, 100 µg/mL | 1st squeeze | 2nd squeeze (cumulative value) | 3rd squeeze (cumulative value) | 4th squeeze (cumulative value) | 5th squeeze (cumulative value) |
| Mean volume extracted (mL) | 0.84 | 0.95 | 0.98 | 1.00 | 1.00 |
| SD | 0.10 | 0.04 | 0.03 | 0.03 | 0.02 |
| RSD (%) | 11.90 | 4.21 | 3.06 | 3.00 | 2.00 |
| 95% CI (mL) | 0.82-0.87 | 0.94-0.97 | 0.98-0.99 | 0.99-1.00 | 0.99-1.00 |

Similar results were obtained with water-alcohol solutions of T3.

Suitable single-dose laminated containers which guarantee the physical and chemical stability of the solution they contain were also identified. The following single-dose laminated containers, filled with the T4 solution (100 µg/mL) described in detail in Example 1, were selected.
1) PE-EVOH-PE70/PP200 ACLAR^{®} laminated container, 400 µm thick, made with a blowing technique
2) PE, EVOH, PP and ACLAR^{®} laminated container, 400 µm thick, made with a blowing technique
3) 5-layer laminated LDPE, EVOH container, adhesive NF408E, 600 µm thick, made with a blowing technique.

These containers are not part of the claimed invention.

The containers were stored for 30 days at 30°C/65% RH. The results of the physical and chemical stability tests on the T4 solution are set out in Table 7.

**Table 7**

| 100 µg/mL of T4 solution was stored at 30°C/65% RH for 30 days | | | | |
|---|---|---|---|---|
| **Prototype** | **Ethanol (%)** | | **Total impurities (%)** | |
| | T=0 | T=30 days | T=0 | T=30 days |
| 1) Laminated PE-EVOH-PE70/PP200 Aclar single-dose container | 99.8 | 93.0 | 0.60 | 0.80 |
| 2) Laminated PE-EVOH-PP-Aclar single-dose container | 99.8 | 96.4 | 0.70 | 1.30 |
| 3) Laminated 5-layer LDPE-EVOH single-dose container, adhesive NF408E | 99.8 | 98.0 | 0.70 | 1.13 |

As demonstrated by the results set out in Table 7, not all the laminated containers proved able to guarantee the chemical stability of the solution they contain. Specifically, the first laminated container does not guarantee suitable resistance to ethanol. The preparation of the second and third prototypes will be more particularly described in the examples below.

The extractability of the water-alcohol T4 solution from the single-dose 5-layer container containing 1.1 mL reported in table 7 (third single-dose container described) was evaluated by way of example.

The process used is similar to the one previously described for the single-dose LDPE container.

The mean values of the volumes squeezed out are shown in Table 8, together with the standard deviation (SD), coefficient of variation (CV%) and 95% confidence interval (95% CI).

The results demonstrate that a volume of 1.0 mL of T4 solution is completely extracted from the containers if they are squeezed 3-4 times. This guarantees the administration of an accurate, reproducible dose to the patient. The volume extracted does not exceed the nominal value of 1 mL if the container is squeezed further.

**Table 8**

| Extractability of T4 solution from 5-layer single-dose containers | | | | | |
|---|---|---|---|---|---|
| Solution T4, 100 µg/mL | 1st squeeze | 2nd squeeze (cumulative value) | 3rd squeeze (cumulative value) | 4th squeeze (cumulative value) | 5th squeeze (cumulative value) |
| Mean volume extracted (mL) | 0.82 | 0.96 | 0.98 | 1.00 | 1.00 |
| SD | 0.10 | 0.03 | 0.02 | 0.01 | 0.01 |
| RSD (%) | 11.65 | 3.25 | 1.55 | 1.43 | 1.49 |
| 95% CI (mL) | 0.80-0.84 | 0.95-0.97 | 0.98-0.99 | 0.99-1.00 | 1.00-1.00 |

Similar results were obtained with water-alcohol solutions of T3.

As will be described in greater detail in the examples below, one-component LDPE containers in sealed sachets, and suitable multi-component laminated containers, proved to guarantee the physical and chemical stability of the formulation of T3 and T4.

### EXAMPLE 1

### Formulation and preparation of water-alcohol solution

Said formulation is used to prepare T4 doses of up to 200 µg, T3 up to 20 µg, and combinations of both active ingredients.

In the typical composition, excipients such as ethanol and glycerol are used, the concentrations of which can range between 5 and 30% w/v for the former and 70 to 95% w/v for the latter, with water as required.

One liter of water-alcohol solution containing 100 µg/mL of T4 is prepared as follows, using the qualitative/quantitative composition listed below:
1) T4 0.105 g
2) ethanol (96%) 243 g
3) glycerol (85%) 861 g

The T4 is solubilized in ethanol in a suitable dissolver, under continuous stirring at ambient temperature. When a clear solution free of visible non-solubilized residues has been obtained, glycerol is added, and a homogenous, clear, colourless solution is obtained under gentle stirring at ambient temperature. The solution is filtered (0.8 µm), and is then ready for filling a suitable primary container.

### EXAMPLE 2

The solution, prepared as described in example 1, is divided between four different one-component single-dose containers with a nominal volume of 1 mL characterized by different materials:
A) Squeezable single-dose PVC076 container (equal to PVC with a thickness of 60 µm);
B) Squeezable single-dose COPO container (EMAA copolymer resin - MFI 9);
C) Squeezable single-dose SURLYN^{®} 2 container (EMAA ionomer resin - partial Na & Zn salt and additives) - MFI 5;
D) Squeezable single-dose LDPE container (600 µm thick).

Only container D, if additionally placed in a sachet according to claim 1, falls under the scope of the present claims.

The Melt Flow Index or Melt Index (MFI) is the index of fluidity of a molten polymer.

### Filling of disposable containers

The prototypes are prepared on a laboratory scale, using an automatic pipette (Gilson P-1000) to fill disposable containers with 1.1 mL of the glycerol-ethanol solution previously described (prepared as described in example 1), after which the containers are sealed with a Pentaseal-lab benchtop sealing machine.

They are then placed in suitable environmental test chambers, and immediately undergo a stability study under ICH conditions; the equipment used is calibrated and instantly monitored for correct operation.

Table 9 below lists the T4 content obtained during a period of three months after preparation, the T3 value and the sum of the impurities, excluding T3, found in the individual single-dose container during the experiment. The values relating to the condition considered most drastic for the product, i.e. 30°C/65% RH, are recorded.

**Table 9**

| 100 µg/mL T4 solution in single-dose containers stored at 30°C/65% RH | | | | | | | | | |
|---|---|---|---|---|---|---|---|---|---|
| T=0 | | | Type of single-dose container | T=1 month | | | T=3 months | | |
| T4 content (%) | T3 content (%) | Total impurities excluding T3 (%) | | T4 content (%) | T3 content (%) | Total impurities excluding T3 (%) | T4 content (%) | T3 content (%) | Total impurities excluding T3 (%) |
| 101.10 | 0.20 | 0.60 | A | 98.40 | 0.24 | 2.47 | 95.30 | 0.65 | 5.54 |
| | | | B | 97.20 | 0.52 | 2.57 | 96.30 | 0.94 | 4.25 |
| | | | C | 99.10 | 0.49 | 1.79 | 94.70 | 0.64 | 5.59 |
| | | | D | 100.80 | 0.20 | 0.60 | 100.10 | 0.30 | 0.60 |

As will be seen, the single-dose LDPE container, compared in absolute terms with the other materials selected and described, proved able to preserve the chemical quality of the product to a sufficient extent according to the parameters considered.

### EXAMPLE 3

Use of single-dose container with a nominal volume of 1 mL made of one-component LDPE plastic contained in a sealed sachet (PET/A1/PE). Characteristics of sachet:
- Stratified film with high gas and light barrier: Polyester 12 µm, A1 9 µm, Polyethylene 50 µm (values to be considered ± 5-6%);
- Oxygen permeability: 0.1-0.2 cc/m²/day
- Water vapor permeability: 0.1-0.2 g/m²/day

The oxygen permeability was measured in accordance with ASTM Standard D-3985.

The water permeability was measured in accordance with ASTM Standard E-398.

### Filling of disposable containers

The prototypes are prepared on a laboratory scale, using an automatic pipette (Gilson P-1000) to fill disposable containers with 1.1 mL of the glycerol-ethanol solution previously described (prepared as described in example 1), after which the containers are sealed with a Pentaseal-lab benchtop sealing machine. Part of the batch is packaged in a hermetically sealed sachet of the type described above.

The sachets are then placed in suitable environmental test chambers, and immediately undergo a stability study under ICH conditions; the equipment used is calibrated and instantly monitored for correct operation.

The samples stored in the protective sachet demonstrate a greater ethanol containment capacity than the unprotected samples.

The product is industrialized by preparing 50 liters of said formulation (as described in example 1). A rotor dissolver of suitable capacity and a semi-automatic Comas Pentafill SA machine for filling single-dose containers (15 strokes per minute) is used. Part of the batch is packaged in a hermetically sealed sachet of the type described, while the remainder is kept in cardboard boxes but not hermetically protected.

Again in this second case, the samples, after being placed in suitable environmental test chambers, immediately undergo a stability study under ICH conditions; the equipment used is calibrated and instantly monitored for correct operation.

Table 10 below lists the residual ethanol values in the individual single-dose container in the sealed sachet, and without the sachet. The values relating to the ICH condition 30°C/65% RH are recorded.

**Table 10**

| 100 µg/mL T4 solution in single-dose containers with and without sachet, stored at 30°C/65% RH | | | | | |
|---|---|---|---|---|---|
| **Prototype** | **Ethanol (%)** | | | | |
| | T=0 | T=3 months | T=6 months | T=9 months | T=12 months |
| T4 solution in single-dose LDPE container in sachet | 96.6 | 97.2 | 96.6 | 96.3 | 97.1 |
| T4 solution in single-dose LDPE container without sachet | 96.6 | 87.3 | 81.2 | 76.7 | 73.3 |

As will be seen, the single-dose LDPE container in the sealed sachet offered the best performance after only three months of the experiment. A year after the start of the experiment, the percentage difference in the ethanol present in the formulation is measurable at around 24%.

Examples 1 and 2 demonstrate the efficacy of the 1 mL single-dose LDPE container stored in a hermetically sealed sachet (PET/A1/PE).

Similar results are obtained by making industrial batches of T4 as described in example 1 with different doses (25, 50 and 75 µg of T4), stored under ICH conditions at 30°C/65% RH and 25°C/60% RH for 12-18 months. The preparations gave excellent results in terms of physical, chemical and microbiological stability (see Tables 11, 12, 13 and 14).

**Table 11**

| T4 Solution **(25 µg/mL)** in single-dose one-component LDPE container contained in hermetically sealed sachet (PET/A1/PE) | | | | | | | | | | | | |
|---|---|---|---|---|---|---|---|---|---|---|---|---|
| **Analysis** | **Product specifications** | | **0** | **30°C ± 2°C / 65% ± 5% R.H.** | | | | **25°C ± 2°C / 60% ± 5% R.H** | | | | |
| | | | | **3 m** | **6 m** | **9 m** | **12 m** | **3 m** | **6 m** | **9 m** | **12 m** | **18 m** |
| Appearance | Clear, colourless or pale yellow solution | | Conforming | Conforming | Conforming | Conforming | Conforming | Conforming | Conforming | Conforming | Conforming | Conforming |
| Specific gravity | 1.107 ± 0.012 g/cm³ | | 1.116 | 1.115 | 1.109 | 1.110 | 1.108 | 1.115 | 1.118 | 1.112 | 1.111 | 1.109 |
| T4 identification | Positive | | Positive | Positive | Positive | Positive | Positive | Positive | Positive | Positive | Positive | Positive |
| T4 content | 95.0-105.0% of theoretical value | | 99.6 | 98.9 | 98.2 | 97.8 | 97.5 | 100.5 | 99.5 | 99.5 | 99.3 | 98.6 |
| Impurities/Degradation products: | Release: | Stability: | | | | | | | | | | |
| - T3 | ≤ 0.2% | ≤ 2.5% | < LOQ | 1.0 | 1.5 | 1.8 | 2.3 | 0.3 | 0.5 | 0.8 | 1.0 | 1.5 |
| - T2 | ≤ 0.1 % | ≤ 0.5% | < LOQ | < LOQ | 0.2 | 0.2 | 0.2 | < LOQ | < LOQ | < LOQ | < LOQ | 0.2 |
| -Triac | ≤ 0.25% | ≤ 1.0% | < LOQ | < LOQ | 0.5 | 0.7 | 1.0 | < LOQ | < LOQ | < LOQ | 0.45 | 0.5 |
| -Tetrac | ≤ 0.3% | ≤ 1.0% | < LOQ | < LOQ | < LOQ | 0.4 | 0.5 | < LOQ | < LOQ | < LOQ | < LOQ | 0.35 |
| -Single unknown impurities | ≤ 0.05% | ≤ 0.5% | < LOQ | < LOQ | 0.2 | 0.2 | 0.5 | < LOQ | < LOQ | < LOQ | 0.15 | 0.25 |
| -Total impurities ≠ T3 | ≤ 1.0% | ≤ 2.5% | 0.7 | 0.7 | 1.2 | 1.5 | 2.2 | 0.7 | 0.7 | 0.7 | 1.0 | 1.3 |
| Ethanol content | 95.0-105.0% of theoretical value | | 102.2 | 100.5 | 101.9 | 102.3 | 102.1 | 103.0 | 101.5 | 101.2 | 101.0 | 100.0 |
| Microbiological tests: | | | Conforming | -- | -- | -- | Conforming | -- | -- | -- | Conforming | -- |
| -TAMC | ≤ 100 CFU/g | | | | | | | | | | | |
| -TYMC | ≤ 10 CFU/g | | | | | | | | | | | |
| *-Escherichia coli* | Absent/g | | | | | | | | | | | |
| LOQ: Limit of quantitation | | | | | | | | | | | | |

**Table 12**

| T4 Solution **(50 µg/mL)** in single-dose one-component LDPE container contained in hermetically sealed sachet (PET/A1/PE) | | | | | | | | | | | | |
|---|---|---|---|---|---|---|---|---|---|---|---|---|
| **Analysis** | **Product specifications** | | **0** | **30°C** ± **2°C** / **65%** ± **5% R.H.** | | | | **25°C** ± **2°C** / **60%** ± **5% R.H** | | | | |
| | | | | **3 m** | **6 m** | **9 m** | **12 m** | **3 m** | **6 m** | **9 m** | **12 m** | **18 m** |
| Appearance | Clear, colourless or pale yellow solution | | Conforming | Conforming | Conforming | Conforming | Conforming | Conforming | Conforming | Conforming | Conforming | Conforming |
| Relative density | 1.107 ± 0.012 g/cm³ | | 1.107 | 1.110 | 1.109 | 1.109 | 1.108 | 1.108 | 1.110 | 1.109 | 1.110 | 1.107 |
| T4 identification | Positive | | Positive | Positive | Positive | Positive | Positive | Positive | Positive | Positive | Positive | Positive |
| T4 content | 95.0-105.0% of theoretical value | | 102.9 | 101.2 | 99.5 | 98.2 | 98.1 | 101.5 | 101.2 | 100.2 | 99.7 | 98.2 |
| Impurities/ Degradation products: | Release: | Stability: | | | | | | | | | | |
| - T3 | ≤ 0.2% | ≤ 2.5% | < LOQ | 1.1 | 1.8 | 2.0 | 2.4 | 0.5 | 1.2 | 1.3 | 1.5 | 2.1 |
| - T2 | ≤ 0.1% | ≤ 0.5% | < LOQ | < LOQ | < LOQ | < LOQ | 0.1 | < LOQ | < LOQ | < LOQ | 0.15 | 0.2 |
| - Triac | ≤ 0.25% | ≤ 1.0% | < LOQ | < LOQ | 0.4 | 0.6 | 0.75 | < LOQ | < LOQ | < LOQ | 0.45 | 0.8 |
| - Tetrac | ≤ 0.3% | ≤ 1.0% | < LOQ | < LOQ | < LOQ | < LOQ | 0.35 | < LOQ | < LOQ | < LOQ | 0.4 | 0.45 |
| - Single unknown impurities | ≤ 0.05% | ≤ 0.5% | < LOQ | < LOQ | 0.2 | 0.2 | 0.3 | < LOQ | < LOQ | < LOQ | 0.2 | 0.35 |
| - Total impurities ≠ T3 | ≤ 1.0% | ≤ 2.5% | 0.7 | 0.7 | 1.0 | 1.3 | 1.5 | 0.7 | 0.7 | 0.7 | 1.2 | 1.8 |
| Ethanol content | 95.0-105.0% of theoretical value | | 102.2 | 101.5 | 103.5 | 102.6 | 102.5 | 103.0 | 101.5 | 101.2 | 101.1 | 99.5 |
| Microbiological tests: | | | Conforming | -- | -- | -- | Conforming | -- | -- | -- | Conforming | -- |
| - TAMC | ≤ 100 CFU/g | | | | | | | | | | | |
| - TYMC | ≤ 10 CFU/g | | | | | | | | | | | |
| - Escherichi a coli | Absent/g | | | | | | | | | | | |
| LOQ: Limit of quantitation | | | | | | | | | | | | |

**Table 13**

| T4 Solution **(75 µg/mL)** in single-dose one-component LDPE container contained in hermetically sealed sachet (PET/A1/PE) | | | | | | | | | | | | |
|---|---|---|---|---|---|---|---|---|---|---|---|---|
| **Analysis** | **Product specifications** | | **0** | **30°C ± 2°C / 65% ± 5% R.H.** | | | | **25°C ± 2°C / 60% ± 5% R.H** | | | | |
| | | | | **3 m** | **6 m** | **9 m** | **12 m** | **3 m** | **6 m** | **9 m** | **12 m** | **18 m** |
| Appearance | Clear, colourless or pale yellow solution | | Conforming | Conforming | Conforming | Conforming | Conforming | Conforming | Conforming | Conforming | Conforming | Conforming |
| Relative density | 1.107 ± 0.012 g/cm³ | | 1.109 | 1.112 | 1.111 | 1.109 | 1.107 | 1.110 | 1.109 | 1.108 | 1.100 | 1.102 |
| T4 identification | Positive | | Positive | Positive | Positive | Positive | Positive | Positive | Positive | Positive | Positive | Positive |
| T4 content | 95.0-105.0% of theoretical value | | 101.5 | 101.2 | 101.1 | 100.1 | 98.1 | 100.4 | 100.2 | 99.8 | 99.4 | 98.2 |
| Impurities/ Degradation products: | Release: | Stability: | | | | | | | | | | |
| - T3 | ≤ 0.2% | ≤ 2.5% | < LOQ | 1.3 | 2.2 | 2.4 | 2.6 | 1.1 | 1.0 | 1.2 | 1.4 | 1.8 |
| - T2 | ≤ 0.1% | ≤ 0.5% | < LOQ | < LOQ | < LOQ | 0.2 | 0.2 | < LOQ | < LOQ | < LOQ | < LOQ | 0.2 |
| - Triac | ≤ 0.25% | ≤ 1.0% | < LOQ | < LOQ | 0.5 | 0.6 | 0.7 | < LOQ | < LOQ | < LOQ | 0.6 | 0.7 |
| - Tetrac | ≤ 0.3% | ≤ 1.0% | < LOQ | < LOQ | 0.4 | 0.4 | 0.4 | < LOQ | < LOQ | < LOQ | 0.4 | 0.5 |
| - Single unknown impurities | ≤ 0.05% | ≤ 0.5% | < LOQ | < LOQ | 0.2 | 0.2 | 0.2 | < LOQ | < LOQ | < LOQ | 0.1 | 0.1 |
| - Total impurities ≠ T3 | ≤ 1.0% | ≤ 2.5% | 0.7 | 0.7 | 1.2 | 1.4 | 1.5 | 0.7 | 0.7 | 0.7 | 1.2 | 1.5 |
| Ethanol content | 95.0-105.0% of theoretical value | | 103.5 | 102.9 | 103.2 | 102.1 | 101.5 | 103.0 | 101.5 | 101.2 | 101.0 | 100.0 |
| Microbiological tests: | | | Conforming | -- | -- | -- | Conforming | -- | -- | -- | Conforming | -- |
| - TAMC | ≤ 100 CFU/g | | | | | | | | | | | |
| - TYMC | ≤ 10 CFU/g | | | | | | | | | | | |
| - Escherichia coli | Absent/g | | | | | | | | | | | |

**Table 14**

| T4 Solution **(100 µg/mL)** in single-dose one-component LDPE container contained in hermetically sealed sachet (PET/A1/PE) | | | | | | | | | | | | |
|---|---|---|---|---|---|---|---|---|---|---|---|---|
| **Analysis** | **Product specifications** | | **0** | **30°C ± 2°C / 65% ± 5% R.H.** | | | | **25°C ± 2°C / 60% ± 5% R.H** | | | | |
| | | | | **3 m** | **6 m** | **9 m** | **12 m** | **3 m** | **6 m** | **9 m** | **12 m** | **18 m** |
| Appearance | Clear, colourless or pale yellow solution | | Conforming | Conforming | Conforming | Conforming | Conforming | Conforming | Conforming | Conforming | Conforming | Conforming |
| Relative density | 1.107 ± 0.012 g/cm³ | | 1.109 | 1.111 | 1.110 | 1.113 | 1.110 | 1.115 | 1.111 | 1.110 | 1.110 | 1.102 |
| T4 identification | Positive | | Positive | Positive | Positive | Positive | Positive | Positive | Positive | Positive | Positive | Positive |
| T4 content | 95.0-105.0% of theoretical value | | 102.9 | 101.5 | 99.9 | 98.2 | 97.6 | 102.5 | 102.4 | 102.1 | 101.5 | 98.2 |
| Impurities/ Degradation products: | Release: | Stability: | | | | | | | | | | |
| - T3 | ≤ 0.2% | ≤ 2.5% | < LOQ | 1.1 | 1.9 | 2.4 | 2.8 | 0.5 | 1.0 | 1.3 | 1.5 | 2.2 |
| - T2 | ≤ 0.1% | ≤ 0.5% | < LOQ | < LOQ | < LOQ | < LOQ | 0.2 | < LOQ | 0.2 | 0.2 | 0.2 | 0.2 |
| - Triac | ≤ 0.25% | ≤ 1.0% | < LOQ | < LOQ | 0.5 | 0.6 | 0.9 | < LOQ | < LOQ | 0.4 | 0.5 | 0.7 |
| - Tetrac | ≤ 0.3% | ≤ 1.0% | < LOQ | < LOQ | < LOQ | < LOQ | 0.4 | < LOQ | < LOQ | < LOQ | 0.4 | 0.4 |
| - Single unknown impurities | ≤ 0.05% | ≤ 0.5% | < LOQ | < LOQ | 0.1 | 0.2 | 0.2 | < LOQ | 0.1 | 0.1 | 0.2 | 0.2 |
| - Total impurities ≠ T3 | ≤ 1.0% | ≤ 2.5% | 0.7 | 0.7 | 1.0 | 1.2 | 1.6 | 0.7 | 0.85 | 0.85 | 1.3 | 1.5 |
| Ethanol content | 95.0-105.0% of theoretical value | | 102.2 | 101.5 | 103.5 | 102.6 | 100.1 | 103.0 | 101.5 | 101.2 | 101.1 | 99.2 |
| Microbiological tests: | | | Conforming | -- | -- | -- | Conforming | -- | -- | -- | Conforming | -- |
| - TAMC | ≤ 100 CFU/g | | | | | | | | | | | |
| - TYMC | ≤ 10 CFU/g | | | | | | | | | | | |
| - Escherichia coli | Absent/g | | | | | | | | | | | |

### EXAMPLE 4 (not part of the claimed invention)

Samples prepared as described in example 2/D are stored in hermetically sealed sachets consisting of:
- triple heat-sealable stratified film made of Polythene 36 µm, A1 8 µm, Paper 51 µm (single film thicknesses to be considered ± 5-6%).

In this case too, after six months' storage under conditions of 30°C-60% RH, the ethanol content in the sample (97.7%), together with the remaining parameters (T4 content: 96.89%; T3 content: 1.05%; sum total of impurities excluding T3: 2.06%), remained at adequate levels.

### EXAMPLE 5 (not part of the claimed invention)

Samples prepared as described in example 2/D are stored in hermetically sealed sachets consisting of:
- triple heat-sealable stratified film made of low-density SURLYN^{®} resin 23.5 µm, A1 12 µm, Paper 51 µm (single film thicknesses to be considered ± 5-6%).

In this case too, after six months' storage under conditions of 30°C-60% RH, the ethanol content in the sample (96.30%), together with the remaining parameters (T4 content: 97.48%; T3 content: 1.06%; sum total of impurities excluding T3: 1.46%), remained at adequate levels.

Filling of disposable containers made of laminated material without protective sachet

### EXAMPLE 6 (not part of the claimed invention)

The prototypes are prepared on a laboratory scale, using an automatic pipette (Gilson P-1000) to fill 5-layer laminated disposable containers with 1.1 mL of the glycerol-ethanol solution previously disclosed (prepared as described in example 1), after which the containers are sealed with a manual laboratory sealing machine.

The container is made in a specific mould by a blowing technique; it is transparent (but can be opacified to protect it from light), has a wall approx. 600 µm thick, and consists of laminated layers of the following materials: LDPE, EVOH, NF 408 adhesive (PE and maleic anhydride); EVOH performs the main function of a barrier to gas permeation. Specifically, PE is Purell PE 1840 H; EVOH is EVAL 101 B.

The samples prepared were stored at the temperature of 30°C and 65% relative humidity for 30 days. The same Table compares the data obtained from samples stored in single-dose laminated containers with PP but without EVOH, and in single-dose LDPE containers with and without protective sachet. The values determined are expressed as percentages, and shown in Table 15.

**Table 15**

| 100 µg/mL T4 solution in single-dose containers stored at 30°C/65% RH for 30 days | | | | |
|---|---|---|---|---|
| **Prototype** | **Ethanol (%)** | | **Total impurities (%)** | |
| | T=0 | T=30 days | T=0 | T=30 days |
| Laminated PE-EVOH single-dose container | 99.8 | 98.0 | 0.70 | 1.13 |
| Laminated PP single-dose container | | 93.0 | | 1.66 |
| Single-layer LDPE single-dose container without protective sachet | | 83.0 | | 3.92 |
| Single-layer LDPE single-dose container with protective sachet | | 99.0 | | 0.80 |

As will be seen, the single-dose laminated container containing PE-EVOH keeps the product in high-quality conditions, and the values measured (impurities and ethanol content) are similar to those obtained with a single-dose one-component LDPE container stored in a hermetically sealed sachet.

### EXAMPLE 7 (not part of the claimed invention)

1.1 mL disposable laminated containers are filled with the glycerol-ethanol solution already described (prepared as shown in example 1), using an automatic pipette (Gilson P-1000).

Said empty single-dose container is made in a special mould. It is obtained by joining 2 symmetrical half-shells generated by a blowing technique similar to the one used to make blister packs. After volumetric filling it is sealed by a manual laboratory sealing machine.

The wall of the single-dose container is approx. 400 µm thick, and consists of layers of the following materials: PE, EVOH, PP, ACLAR^{®}.

The samples prepared were stored at the temperature of 30°C and 65% relative humidity for 30 days. The same Table compares the data obtained from samples stored in single-dose LDPE containers with and without protective sachet. The values determined are expressed as percentages, and shown in Table 16.

**Table 16**

| 100 µg/mL T4 solution in single-dose containers stored at 30°C/65% RH for 30 days | | | | |
|---|---|---|---|---|
| **Prototype** | **Ethanol (%)** | | **Total impurities (%)** | |
| | T=0 | T=30 days | T=0 | T=30 days |
| Laminated PE-EVOH-PP-Aclar single-dose container | 99.8 | 96.4 | 0.70 | 1.30 |
| Single-layer LDPE single-dose container without protective sachet | | 83.0 | | 3.92 |
| Single-layer LDPE single-dose container with protective sachet | | 99.0 | | 0.80 |

As will be seen, the single-dose laminated container containing PE-EVOH-PP-ACLAR^{®} only 400 µm thick keeps the product in good quality conditions, and the values measured (impurities and ethanol content) are not very different from those obtained with a single-dose one-component LDPE container stored in a hermetically sealed sachet.

The sample subjected to said conditions for 6 months proved stable in terms of general quality. The percentage values found are set out below.
- T4 content: 97.56%;
- Total impurities: 2.44%;
- Ethanol content: 97%.

### EXAMPLE 8

1.1 mL disposable laminated containers are filled with the glycerol-ethanol solution already disclosed (prepared as described in example 1), using an automatic pipette (Gilson P-1000).

Said empty single-dose container is made in a special mould. It is obtained by joining 2 symmetrical half-shells generated by a blowing technique similar to the one used to make blister packs. After volumetric filling it is sealed by a manual laboratory sealing machine.

The wall of the single-dose container is approx. 300 µm thick, and consists of layers of the following materials: PVC, PVDC, PE.

The samples prepared were stored at the temperature of 30°C and 65% relative humidity for 30 days. The same Table compares the data obtained from samples stored in single-dose LDPE containers with and without protective sachet. The single-dose LDPE container without protective sheet is not according to the present invention. The values determined are expressed as percentages, and shown in Table 17.

**Table 17**

| 100 µg/mL T4 solution in single-dose containers stored at 30°C/65% RH for 30 days | | | | |
|---|---|---|---|---|
| **Prototype** | **Ethanol (%)** | | **Total impurities (%)** | |
| | T=0 | T=30 days | T=0 | T=30 Days |
| Laminated single-dose PVC-PVDC-PE container | 99.8 | 97.8 | 0.70 | 1.20 |
| Single-layer LDPE single-dose container without protective sachet | | 83.0 | | 3.92 |
| Single-layer LDPE single-dose container with protective sachet | | 99.0 | | 0.80 |

As will be seen, the single-dose laminated container containing PVC, PVDC, PE only 300 µm thick keeps the product in good quality conditions, and the values measured (impurities and ethanol content) are not very different from those obtained with a single-dose one-component LDPE container stored in a hermetically sealed sachet.

The sample subjected to said conditions for 6 months proved stable in terms of general quality. The percentage values found are set out below.
- T4 content: 97.54%;
- Total impurities: 2.46%;
- Ethanol content: 98%.

## Claims

1. A single-dose pharmaceutical preparation of T3 and T4 thyroid hormones suitable for oral administration, in ready-to-use packaging consisting of a container pre-filled with a water-alcohol solution of hormone T3 and/or T4, said container being selected from:
(a) a one-component LDPE plastic container, squeezable by hand-compression and having a Young's modulus of between 10 and 200 MPa, placed in a sealed sachet consisting of a laminated film made of different materials, in the following combination: polyethylene, aluminium and polyester.
(b) a multi-component laminated plastic container having a thickness of between 200 and 700 µm, squeezable by hand-compression and having a Young's modulus of between 10 and 200 MPa, said container consisting of multiple layers of the following plastic materials: polyvinyl chloride, polyvinylidene chloride and polyethylene.

2. A pharmaceutical preparation according to claim 1, in ready-to-use packaging according to claim 1, wherein said sachet exhibits oxygen and water-vapor permeability ranging from 0.1 to 0.2 cc/m2/day.

3. A pharmaceutical preparation according to claims 1-2, in ready-to-use packaging according to claims 1-2, wherein the water-alcohol solution contains 1 to 20 µg T3 thyroid hormone and/or 12.5 to 200 µg T4 thyroid hormone.

4. A pharmaceutical preparation according to claim 3, in ready-to-use packaging according to claim 3, wherein said water-alcohol solution also contains ethyl alcohol, glycerol and water.

5. A pharmaceutical preparation according to claim 4, in ready-to-use packaging according to claim 4, wherein said water-alcohol solution contains 5 to 30% w/v ethyl alcohol and 70 to 95% w/v glycerol.

6. A pharmaceutical preparation according to claims 1-5, in ready-to-use packaging according to claims 1-5, for use in the treatment of diseases associated with T3 and/or T4 hormone deficiency.

7. The use of a water-alcohol solution of T3 and/or T4 thyroid hormone according to claims 1-5, in a container according to claims 1-5, for the preparation of a medicament for oral administration to be used in the treatment of diseases associated with T3 and/or T4 hormone deficiency.

## Patentansprüche

1. Pharmazeutisches Einzeldosis-Präparat aus den Schilddrüsenhormonen T3 und T4, welches für die orale Anwendung geeignet ist, in gebrauchsfertigen Verpackungen bestehend aus einem Behälter, welcher mit einer Wasser-Alkohollösung der Hormone T3 und/oder T4 vorgefüllt ist, wobei der Behälter ausgewählt ist aus:
(a) ein Behälter, welcher aus einem Einkomponenten-Kunststoff LDPE hergestellt ist, durch händisches Zusammenpressen zusammendrückbar ist und ein Elastizitätsmodul zwischen 10 und 200 MPa aufweist, welcher sich in einem abgedichteten Beutel befindet, bestehend aus einem aus verschiedenen Materialien bestehenden laminierten Film, in der folgenden Zusammenstellung: Polyethylen, Aluminium und Polyester.
(b) ein Behälter, welcher aus laminiertem Mehrkomponenten-Kunststoff hergestellt ist, eine Dicke zwischen 200 und 700 µm aufweist, durch händisches Zusammenpressen zusammendrückbar ist und ein Elastizitätsmodul zwischen 10 und 200 MPa aufweist, wobei der Behälter aus mehreren Schichten von folgenden Kunststoffmaterialien besteht: Polyvinylchlorid, Polyvinylidenchlorid und Polyethylen.

2. Pharmazeutisches Präparat nach Anspruch 1, in einer gebrauchsfertigen Verpackung nach Anspruch 1, wobei der Beutel eine Sauerstoff- und Wasserdampfpermeabilität von 0,1 bis 0,2 cc/m2/Tag aufweist.

3. Pharmazeutisches Präparat nach Ansprüchen 1-2, in einer gebrauchsfertigen Verpackung nach Ansprüchen 1-2, wobei die Wasser-Alkohollösung 1 bis 20 µg T3 Schilddrüsenhormon und/oder 12,5 bis 200 µg T4 Schilddrüsenhormon beinhaltet.

4. Pharmazeutisches Präparat nach Anspruch 3, in einer gebrauchsfertigen Verpackung nach Anspruch 3, wobei die Wasser-Alkohollösung auch Ethylalkohol, Glyzerin und Wasser beinhaltet.

5. Pharmazeutisches Präparat nach Anspruch 4, in einer gebrauchsfertigen Verpackung nach Anspruch 4, wobei die Wasser-Alkohollösung 5 bis 30 % w/v Ethylalkohol und 70 bis 95 % w/v Glyzerin beinhaltet.

6. Pharmazeutisches Präparat nach Ansprüchen 1-5, in einer gebrauchsfertigen Verpackung nach Ansprüchen 1-5, zur Verwendung in der Behandlung von Krankheiten, welche mit T3 und/oder T4 Hormonmangel assoziiert sind.

7. Verwendung einer Wasser-Alkohollösung mit T3 und/oder T4 Schilddrüsenhormonen nach Ansprüchen 1-5, in einem Behälter gemäß Ansprüchen 1-5, für die Herstellung eines Medikaments für die orale Anwendung, um bei der Behandlung von Krankheiten, welche mit T3 und/oder T4 Hormonmangel assoziiert sind, verwendet zu werden.

## Revendications

1. Préparation pharmaceutique monodose d'hormones thyroïdiennes T3 et T4 appropriée pour une administration orale, dans un emballage prêt à l'emploi constitué d'un récipient prérempli avec une solution hydroalcoolique d'hormone T3 et/ou T4, ledit récipient étant choisi parmi:
(a) un récipient en matière plastique LDPE à un seul composant, apte à être pressé par compression à la main et ayant un module d'Young entre 10 et 200 MPa, placé dans un sachet scellé constitué d'un film stratifié fait de différents matériaux, dans la combinaison suivante: polyéthylène, aluminium et polyester
(b) un récipient en matières plastiques stratifié à composants multiples ayant une épaisseur entre 200 et 700 µm, apte à être pressé par compression à la main et ayant un module d'Young entre 10 et 200 MPa, ledit récipient étant constitué de multiples couches des matières plastiques suivants: le polychlorure de vinyle, le polychlorure de vinylidène et le polyéthylène.

2. Préparation pharmaceutique selon la revendication 1, dans un emballage prêt à l'emploi selon la revendication 1, où ledit sachet présente une perméabilité à l'oxygène et à la vapeur d'eau se situant dans une plage allant de 0,1 à 0,2 cc/m²/jour.

3. Préparation pharmaceutique selon les revendications 1 et 2, dans un emballage prêt à l'emploi selon les revendications 1 et 2, où la solution hydroalcoolique contient 1 à 20 µg d'hormone thyroïdienne T3 et/ou 12,5 à 200 µg d'hormone thyroïdienne T4.

4. Préparation pharmaceutique selon la revendication 3, dans un emballage prêt à l'emploi selon la revendication 3, où ladite solution hydroalcoolique contient également de l'alcool éthylique, du glycérol et de l'eau.

5. Préparation pharmaceutique selon la revendication 4, dans un emballage prêt à l'emploi selon la revendication 4, où ladite solution hydroalcoolique contient 5 à 30 % p/v d'alcool éthylique et 70 à 95 % p/v de glycérol.

6. Préparation pharmaceutique selon les revendications 1 à 5, dans un emballage prêt à l'emploi selon les revendications 1 à 5, pour une utilisation dans le traitement de maladies associées à un déficit en hormone T3 et/ou T4.

7. Utilisation d'une solution hydroalcoolique d'hormone thyroïdienne T3 et/ou T4 selon les revendications 1 à 5, dans un récipient selon les revendications 1 à 5, pour la préparation d'un médicament pour une administration orale à utiliser dans le traitement de maladies associées à un déficit en hormone T3 et/ou T4.
